# EUROPÄISCHE PATENTSCHRIFT

(11) **EP 3 657 442 B1**
(45) Veröffentlichungstag und Bekanntmachung des Hinweises auf die Patenterteilung: **26.10.2022**
(21) Anmeldenummer: 18214794.2
(22) Anmeldetag: 20.12.2018
(51) Int. Cl.: G06T 11/00, G06T 15/08

(54) **SYNTHETISCHES MAMMOGRAMM MIT AUFGEPRÄGTEM BILDEINDRUCK**
SYNTHETIC MAMMOGRAMM WITH EMBOSSED IMAGE IMPRESSION
MAMMOGRAMME SYNTHÉTIQUE À IMPRESSION ESTAMPÉE D'IMAGE

(30) Priorität: 23.11.2018 EP 18208120
(43) Veröffentlichungstag der Anmeldung: 27.05.2020
(73) Patentinhaber: Siemens Healthcare GmbH, 91052 Erlangen (DE)
(72) Erfinder: WICKLEIN, Julia, Dr., 91077 Neunkirchen a. Br. (DE); HE, Wen Man, 91054 Erlangen (DE); RITSCHL, Ludwig, Dr., 91054 Erlangen (DE); BINIAZAN, Ramyar, 90402 Nürnberg (DE); DWARS, Stephan, Dr., 90489 Nürnberg (DE)

(56) Entgegenhaltungen:
- WO-A1-2016/078958
- US-A1- 2018 185 000

## Beschreibung

Die Erfindung betrifft die Erzeugung eines synthetischen Mammogramms mit aufgeprägtem Bildeindruck.

Digitale Brusttomosynthese (DBT) erlaubt eine dreidimensionale Bildgebung der Brust. Mehrere Schichten in unterschiedlichen Positionen, insbesondere Höhen, der Brust werden aus einer Vielzahl von Projektionsdatensätzen, beispielsweise 25, rekonstruiert. Die Aufnahme eines Projektionsdatensatzes findet unter einem Projektionswinkel statt. Die Projektionsdatensätze werden für verschiedene Projektionswinkel aufgenommen. Die verschiedenen Projektionswinkel können insbesondere in einem beschränkten Winkelbereich von beispielsweise 50 Grad aufgenommen werden.

Ein großer Vorteil von digitaler Brusttomosynthese gegenüber einer konventionellen digitalen Vollfeld-Mammographie (engl.: full-field digital mammography, FFDM) ist die Möglichkeit überlappende Gewebe aufzulösen. Dies ist insbesondere vorteilhaft für die Identifikation sogenannter Massen mit spikulierte Läsionen (engl.: spiculated lesions) in bestimmten Schichten, welche in einer digitalen Vollfeld-Mammographieaufnahme durch überlappende Gewebestrukturen oder Gefäßen anderer Schichten überlagert sein können und damit nur schwer erkennbar sind. Dennoch hat die digitale Vollfeld-Mammographie einen Vorteil, wenn es um die Geschwindigkeit bei der Bildbetrachtung und der Darstellung von Mikrokalzifikationsclustern geht. Daher umfassen klinische Protokolle derzeit die digitale Brusttomosynthese und die digitale Vollfeld-Mammographie, um die Vorteile beider Bildgebungstechniken zu kombinieren. Nachdem für die digitale Brusttomosynthese und die digitale Vollfeld-Mammographie jeweils eine ähnliche Patientendosis verwendet wird, wird für die Kombination beider Bildgebungstechniken in etwa die doppelte Dosis im Vergleich zur alleinigen digitalen Vollfeld-Mammographie nötig. Daher ist es wünschenswert aus den Aufnahmedatensätzen bzw. Projektionsdatensätzen der digitalen Brusttomosynthese ein synthetisches Mammogramm zu berechnen, wobei die zusätzliche Dosis vermieden werden kann und die Vorteile der zweidimensionalen digitalen Vollfeld-Mammographie erhalten bleiben. Eine spezielle Anwendung für eine derartige zweidimensionale Darstellung ist der Vergleich mit früheren Aufnahmen. Diese früheren Aufnahmen sind digitale Vollfeld-Mammographieaufnahmen früherer Screenings oder Untersuchungen, welche für den betreffenden Patienten bzw. die betreffende Patientin gespeichert wurden. Nun ist es die Aufgabe, Unterschiede zwischen den aktuellen und vorherigen Gegebenheiten bzw. Auffälligkeiten und deren Größenverhältnissen innerhalb der Brust zu finden. Beispielsweise wird die Verteilung dichten Brustgewebes oder das Wachstum spezifischer Massen betrachtet und verglichen. Kunden bzw. Benutzer können für die Nachverarbeitung der digitalen Vollfeld-Mammographieaufnahme zwischen unterschiedlichen vordefinierten Bildeindruckseinstellungen, sogenannten Flavours, wählen.

Die Berechnung des synthetischen Mammogramms bzw. einer synthetischen Projektion auf Basis von Tomosynthesedatensätzen bringt jedoch einige technische Hürden mit sich. Nachdem die Projektionsdatensätze nur mit einem Bruchteil der Dosis für eine digitale Vollfeld-Mammographieaufnahme aufgenommen werden, beispielsweise 1/25 der Dosis bei 25 Projektionen, leidet das Kontrast-zu-Rausch-Verhältnis enorm für jede einzelne Projektion. Zudem kann mit einer Verschmierung der Information aufgrund der Bewegung der Röntgenquelle gerechnet werden. Daher erfüllt die Verwendung einer einzigen Projektionsaufnahme nicht die Anforderungen an eine Aufnahme in zufriedenstellender Qualität. Rückprojektionen der rekonstruierten Volumendaten mit einer Durchschnitts-Intensitäts-Projektion (engl.: average intensity projection, AIP), welche einer physikalischen Linienintegration ähnelt leidet ebenfalls unter einer Verschmierung von Informationen. Dies kann Rekonstruktionsartefakten zugeschrieben werden, wie es beispielsweise in den Druckschriften EP 2 998 936 B1 und US 9 569 864 B2 beschrieben wird.

Aus der Druckschrift EP 2 998 936 B1 ist ein Verfahren zum Erzeugen eines kombinierten Projektionsbildes von einem medizinischen Inspektionsobjekt bekannt, welches die folgenden Schritte umfasst:
- Erfassen einer Menge von Anfangsprojektionsbildern aus verschiedenen Projektionsrichtungen;
- Rekonstruieren eines ersten und eines zweiten dreidimensionalen Volumens aus einer reduzierten Teilmenge der Anfangsprojektionsbilder, welche eine Anzahl von Anfangsprojektionsbildern aus Blickpositionen umfasst, die einer Reprojektions-Blickposition benachbart sind;
- Erzeugen eines ersten Reprojektionsbildes aus dem ersten dreidimensionalen Volumen und eines zweiten Reprojektionsbildes aus dem zweiten dreidimensionalen Volumen, wobei das erste und das zweite Reprojektionsbild unter Verwendung der Reprojektions-Blickposition erzeugt werden;
- Gewichten des ersten Reprojektionsbildes und des zweiten Reprojektionsbildes; und
- Kombinieren des gewichteten ersten Reprojektionsbildes und zweiten Reprojektionsbildes zum Erzeugen des kombinierten Projektionsbildes.

Aus der Druckschrift US 9 569 864 B2 ein Verfahren zur Erzeugung eines Projektionsbildes aus tomographischen Bildern bekannt, welches die folgenden Schritte umfasst:
- Aufnahme einer Mehrzahl von gestapelten zweidimensionalen tomographischen Bildern, welche ein tomographisches Volumen darstellen;
- Wichtung der Pixel der gestapelten zweidimensionalen tomographischen Bilder entlang einer Anzahl von Projektionsstrahlen durch das tomographische Volumen mit Wichtungsfaktoren, welche mit der Einschränkung gewählt wurden, dass die Summe aller quadrierten Wichtungsfaktoren für jede individuelle Projektion zwischen einer gegebenen unteren Grenze und einer gegebenen oberen Grenze liegen, wobei die gegebene untere Grenze gleich der gegebenen oberen Grenze gesetzt ist; und
- Erzeugen eines zweidimensionalen Projektionsbildes durch Aufsummieren der gewichteten Pixel entlang der Anzahl an Projektionen.

Aus der Druckschrift US 2018/185000 A1 ist eine Steuereinheit bekannt, welche einen Einfallswinkel von Strahlung, die auf eine Detektionsfläche eines Strahlungsdetektors einfällt, in Bezug auf die Detektionsfläche auf eine Vielzahl von voneinander verschiedenen Winkeln einstellt, einschließlich eines Winkels (0 Grad) in einer senkrechten Richtung der Detektionsfläche. Diese erfasst mindestens eines einer Vielzahl von Projektionsbildern, die von dem Strahlungsdetektor in Übereinstimmung mit dem Einfallswinkel erfasst werden, oder rekonstruierte Bilder, die unter Verwendung der Projektionsbilder rekonstruiert werden. Zusätzlich erzeugt die Steuereinheit auf der Grundlage des einen Bildes ein erstes synthetisches Mammogrammbild. Zusätzlich erzeugt die Steuereinheit ein zweites synthetisches Mammogrammbild, indem sie ein Hochfrequenzbild mit einer Hochfrequenzkomponente, die höher als eine vorbestimmte Frequenz des ersten synthetischen Mammogrammbildes ist, und ein Niederfrequenzbild mit einer Niederfrequenzkomponente, die gleich oder niedriger als eine vorbestimmte Frequenz eines Null-Grad-Projektionsbildes ist, synthetisiert.

Aus der Druckschrift WO 2016/078958 A1 ist ein Verfahren und eine zugehörige Vorrichtung zum Synthetisieren eines Projektionsbildes, insbesondere zur Verwendung in der Mammographie, bekannt. Es wird vorgeschlagen, aus einem Bildvolumen eine Gewichtsfunktion zu berechnen, die dann dazu verwendet wird, eine gewichtete Vorwärtsprojektion durch einen anderen Bildvolumenblock zu implementieren, um ein synthetisiertes Projektionsbild über den Block zu berechnen.

Der Erfindung liegt das Problem zugrunde, dass synthetische Mammogramme jedoch in einem von der digitalen Vollfeld-Mammographieaufnahme verschiedenen Dynamikbereich berechnet werden und mittels einer von der digitalen Vollfeld-Mammographieaufnahme verschiedenen Nachbearbeitung bearbeitet werden. Die Verschiedenheit des Dynamikbereichs ergibt sich aus den Berechnungsmethoden des synthetischen Mammogramms. Dadurch wird bisher nur eine einzige Bildeindruckseinstellung bzw. ein einziger Bildeindruck für alle Benutzer bereitgestellt, welche sich vom Bildeindruck bzw. hinsichtlich mindestens einer Bildeigenschaft einer digitalen Vollfeld-Mammographieaufnahme unterscheidet. Der Vergleich früherer digitaler Vollfeld-Mammographieaufnahmen mit aktuellen synthetischen Mammogrammen wird dadurch erheblich erschwert, insbesondere für Benutzer mit sehr speziellen bzw. einzigartigen Bildeindruckseinstellungen der digitalen Vollfeld-Mammographieaufnahmen.

Es ist Aufgabe der Erfindung, ein Verfahren und eine Vorrichtung anzugeben, welche einen Bildeindruck entsprechend einer früheren digitalen Vollfeld-Mammographieaufnahme ermöglichen.

Die Aufgabe wird erfindungsgemäß gelöst durch ein Verfahren zum Erzeugen eines synthetischen Mammogramms nach Anspruch 1 und ein Mammographiesystem nach Anspruch 11.

Die Erfindung betrifft ein Verfahren zum Erzeugen eines synthetischen Mammogramms aufweisend die Schritte des Aufnehmens und des Erzeugens. Ein synthetisches Mammogramm kann insbesondere einen zweidimensionalen Bilddatensatz bezeichnen, welcher eine der konventionellen digitalen Vollfeld-Mammographieaufnahme im Wesentlichen entsprechende Ansicht des Untersuchungsobjekts umfasst. Das synthetische Mammogramm wird dabei insbesondere aus einem im Wesentlichen dreidimensionalen Datensatz, insbesondere mehreren Projektionsdatensätzen erzeugt. Im Schritt des Aufnehmens wird eine Mehrzahl von Projektionsdatensätzen unter einer Mehrzahl von Projektionswinkeln aufgenommen. Die Projektionswinkel liegen in einem begrenzten Winkelbereich. Der Winkelbereich kann im Bereich zwischen 40 und 90 Grad liegen, bevorzugt entspricht der Winkelbereich in etwa 50 Grad. Die Anzahl der Projektionswinkel kann im Bereich zwischen 15 und 40 liegen, bevorzugt werden 25 Projektionen aufgenommen. Die Mehrzahl der Projektionsdatensätze kann insbesondere mit einem Röntgenspektrum aufgenommen werden. Die verwendete Dosis bzw. der verwendete Röhrenstrom kann für die Mehrzahl der Projektionsdatensätze im Wesentlichen gleich sein. Im Schritt des Erzeugens wird mindestens ein synthetisches Mammogramm mit zu einer konventionellen digitalen Vollfeld-Mammographieaufnahme im Wesentlichen äquivalenten Bildeigenschaft basierend auf mehreren Projektionsdatensätzen erzeugt. Im Schritt des Erzeugens kann eine Auswahl aus der Mehrzahl von Projektionsdatensätzen oder alle Projektionsdatensätze verwendet werden.

Die äquivalente Bildeigenschaft kann insbesondere dazu führen, dass der Bildeindruck des synthetischen Mammogramms im Wesentlichen dem Bildeindruck einer konventionellen digitalen Vollfeld-Mammographieaufnahme entspricht. Zudem kann der Dynamikbereich des synthetischen Mammogramms im Wesentlichen dem Dynamikbereich, dem Kontrast oder/und der Helligkeit einer konventionellen digitalen Vollfeld-Mammographieaufnahme entsprechen. Die Bildeigenschaft kann den Dynamikbereich, den Kontrast oder die Helligkeit umfassen. Die äquivalente Bildeigenschaft kann bedeuten, dass die Bildeigenschaft des synthetischen Mammogramms und die Bildeigenschaft der konventionellen digitalen Voll-Mammographieaufnahme im Wesentlichen gleich ist. Die Bildeigenschaft kann sich beispielsweise um wenige Prozent, beispielsweise weniger als 10 Prozent, bevorzugt weniger als 5 Prozent unterscheiden. Vorteilhaft kann ein Vergleich des synthetischen Mammogramms mit einer früheren konventionellen digitalen Vollfeld-Mammographieaufnahme erheblich vereinfacht werden. Beispielsweise kann das synthetische Mammogramm eine aufgeprägte, ausgewählte vordefinierte Bildeindruckseinstellung, sogenannter Flavour, umfassen, wobei die Bildeindruckseinstellung derart ausgewählt ist, dass sie der vordefinierten Bildeindruckseinstellung der früheren konventionellen digitalen Vollfeld-Mammographieaufnahme entspricht.

Im Schritt des Erzeugens wird ein synthetisches Mammogramm erzeugt oder es werden mehrere synthetische Mammogramme erzeugt. Bevorzugt wird zumindest ein synthetisches Mammogramm entsprechend des mittleren Projektionsdatensatzes, insbesondere bei einem Projektionswinkel von 0 Grad, erzeugt. Der mittlere Projektionswinkel kann dabei insbesondere den Winkel bezeichnen, bei dem der Zentralstrahl der Röntgenquelle im Wesentlichen senkrecht auf die komprimierte Brust bzw. senkrecht auf das obere Kompressionselement einfällt. Bevorzugt können mehrere synthetische Mammogramme erzeugt werden. Ein synthetisches Mammogramm kann jeweils einem Projektionswinkel zugeordnet werden. Es kann für einen Projektionswinkel ein synthetisches Mammogramm erzeugt werden. Die maximale Anzahl der erzeugten synthetischen Mammogramme kann beispielsweise der Anzahl der Projektionswinkel entsprechen. Bevorzugt wird ein synthetisches Mammogramm für den Projektionswinkel 0 Grad erzeugt. Weiterhin werden 2 bis zur maximalen Anzahl der aufgenommenen Projektionswinkel, bevorzugt 10 bis 20, besonders bevorzugt 17, synthetische Mammogramme erzeugt. Das synthetische Mammogramm kann insbesondere auf der Projektionsaufnahme des zugeordneten Projektionswinkels basieren, beispielsweise unter Verwendung dieser Projektionsaufnahme in Form einer Durchschnittsintensitätsprojektion. Beispielsweise werden 17 synthetische Mammogramme basierend auf 25 Projektionsdatensätzen erzeugt.

Die Erfinder haben erkannt, dass es für das Ermöglichen einer Verwendung einer Standard-Nachverarbeitung für digitale Vollfeld-Mammographieaufnahmen nötig ist, ein neuartiges synthetisches Mammogramm zu berechnen, welches äquivalent zu einer Detektorausgabe ist. Das synthetische Mammogramm, welches äquivalent zu einer Detektorausgabe ist, weist mindestens eine, bevorzugt mehrere, im Wesentlichen gleiche Bildeigenschaft auf. Dieses synthetische Mammogramm muss daher idealerweise im gleichen dynamischen Bereich von konventionellen digitalen Vollfeld-Mammographieaufnahmen liegen. Durch das erfindungsgemäße Verfahren und dem Zusammenwirken der davon umfassten Schritte kann dies erreicht werden. Nach der Rekombination der unterschiedlichen Bildkomponenten der unterschiedlichen Schritte zu einem synthetischen Mammogramm kann vorteilhaft die gleiche Nachverarbeitung wie für digitale Vollfeld-Mammographieaufnahme für das synthetische Mammogramm verwendet werden.

Das Mammographiesystem umfasst eine Röntgenquelle und einen Röntgendetektor sowie eine Kompressionseinheit. Insbesondere die Röntgenquelle ist bezüglich des Röntgendetektors sowie der Kompressionseinheit rotierbar gelagert, dadurch sind verschiedene Projektionswinkel einstellbar. Die Kompressionseinheit umfasst ein oberes Kompressionselement und ein unteres Kompressionselement, zwischen denen die Brust eines Patienten angeordnet wird. Im Schritt des Aufnehmens werden Projektionsdatensätze mit der Brust als Untersuchungsobjekt unter verschiedenen Projektionswinkeln aufgenommen, während die Brust zwischen dem oberen und dem unteren Kompressionselement komprimiert wird und im während des Schrittes des Aufnehmens im Wesentlichen unverändert komprimiert bleibt.

Vorteilhaft kann das synthetische Mammogramm mit einer früheren digitalen Vollfeld-Mammographieaufnahme verglichen werden. Der Vergleich findet bevorzugt optisch statt. Beispielsweise können das synthetische Mammogramm und die frühere digitale Vollfeld-Mammographieaufnahme nebeneinander angezeigt werden. Ferner kann ein Links-Rechts-Vergleich von synthetischen Mammogrammen und der früheren digitale Vollfeld-Mammographieaufnahmen nebeneinander ermöglicht werden. Vorteilhaft kann die Kombination bzw. die kombinierte Aufnahme von digitaler Vollfeld-Mammographieaufnahme und digitaler Brusttomosynthese nacheinander vermieden werden, damit kann vorteilhaft die Patientendosis reduziert werden. Vorteilhaft kann die Brustdichte besonders einfach erfasst werden. Vorteilhaft kann die Patientendosis reduziert werden. Vorteilhaft kann die Zeit zur Bewertung der Aufnahmen verkürzt werden, da eine zusätzliche aktuelle konventionelle digitale Vollfeld-Mammographieaufnahme vermieden werden kann.

Gemäß einem Aspekt der Erfindung weist das ferner den Schritt des Aufprägens auf, wobei eine ausgewählte vordefinierte Bildeindruckseinstellung auf das synthetische Mammogramm aufgeprägt wird. Mittels der Aufprägung der ausgewählten vordefinierten Bildeindruckseinstellung kann der Bildeindruck des synthetischen Mammogramms angepasst bzw. verändert werden. Die Bildeindruckseinstellung kann als sogenannter Flavour bezeichnet werden. Die Bildeindruckseinstellung kann beispielsweise aus einer der folgenden Varianten ausgewählt werden: Standard, geglättet, Kontrast-verstärkt, Kanten-verstärkt, oder Kontrast-und-Kanten-verstärkt. Vorteilhaft kann der Bildeindruck vom Benutzer angepasst werden.

Gemäß einem Aspekt der Erfindung umfasst die vordefinierte Bildeindruckseinstellung eine Multifrequenzanpassung, eine Lookup-Tabellen-Anwendung, oder/und eine Fensterung. Vorteilhaft kann der Bildeindruck reproduzierbar aufgeprägt werden.

Erfindungsgemäß umfasst der Schritt des Erzeugens ein Ermitteln einer Durchschnittsintensitätsprojektion (AIP) basierend auf der Mehrzahl von Projektionsdatensätzen als erste Bildkomponente. Bevorzugt kann ein Projektionsdatensatz eines Projektionswinkels, insbesondere entsprechend der Zuordnung eines Projektionswinkels zum synthetischen Mammogramm, als Durchschnittsintensitätsprojektion oder als Basis für die Durchschnittsintensitätsprojektion verwendet werden. Insbesondere kann eine limited-angle-AIP (LARIP) als Durchschnittsintensitätsprojektion verwendet werden. Es kann ein Projektionsdatensatz oder es können mehrere Projektionsdatensätze ausgewählt werden, auf deren Basis die Durchschnittsintensitätsprojektion ermittelt wird. Vorteilhaft umfasst die Durchschnittsintensitätsprojektion als erste Bildkomponente wesentliche Informationen über das Untersuchungsobjekt in der zweidimensionalen Draufsicht entsprechend des Projektionswinkels. Die Durchschnittsintensitätsprojektion kann alleine jedoch kaum den Anforderungen für eine umfassende Beurteilung der Brust genügen, da sie aufgrund der nur anteilig verwendeten Dosis ein relativ hohes Rauschen aufweist.

Erfindungsgemäß wird auf die Durchschnittsintensitätsprojektion zumindest einer der folgenden Schritte angewendet: Intensitätsanpassung, Grauwertverteilungsanpassung, Streustrahlenkorrektur, und kalkerhaltender Rauschfilter. Die Durchschnittsintensitätsprojektion kann derart bearbeitet werden, dass die bearbeitete Durchschnittsintensitätsprojektion als rauschige Basis bzw. erste Bildkomponente für das synthetische Mammogramm verwendet werden kann. Vorteilhaft kann die Information dieser Projektion als Grundlage für das synthetische Mammogramm dienen. Auf die erste Bildkomponente aufbauend kann durch Hinzufügen von Kanteninformationen und Kontrastinformationen einer zweiten Bildkomponente ein zu einer konventionellen digitalen Vollfeld-Mammographieaufnahme im Wesentlichen qualitativ äquivalentes synthetisches Mammogramm erzeugt werden.

Erfindungsgemäß umfasst der Schritt des Erzeugens ein Ermitteln einer Maximumsintensitätsprojektion (MIP) basierend auf der Mehrzahl von Projektionsdatensätzen als zweite Bildkomponente. Es können insbesondere mehrere Projektionsdatensätze zum Ermitteln der Maximumsintensitätsprojektion verwendet werden. Es können insbesondere Projektionsdatensätze verwendet werden, welche benachbart sind zu dem synthetischen Mammogramm zugeordneten Projektionsdatensatz bzw. deren Projektionswinkel. Beispielsweise können Projektionsdatensätze eines geeigneten Winkelbereichs um den zugeordneten Projektionswinkel verwendet werden. Es können insbesondere alle Projektionsdatensätze zum Ermitteln der Maximumsintensitätsprojektion verwendet werden. Vorteilhaft können Kanteninformationen und Kontrastinformationen aus mehreren Projektionsdatensätzen als zweite Bildkomponente genutzt werden, um diese der ersten Bildkomponente hinzuzufügen. Vorteilhaft kann die zweite Bildkomponente zum Entrauschen verwendet werden.

Gemäß einem Aspekt der Erfindung wird die Maximumsintensitätsprojektion in mindestens zwei verschiedene Frequenzkomponenten zerlegt. Es kann eine Frequenzbandzerlegung durchgeführt werden. Gemäß einem Aspekt der Erfindung umfassen die mindestens zwei verschiedenen Frequenzkomponenten hochfrequente Komponenten und mittelfrequente Komponenten. Die mindestens zwei Frequenzkomponenten können mit oder ohne Überlapp zwischen einander ausgebildet sein. Mittels der hochfrequenten Komponente können die Kanten besonders hervorgehoben werden. Mittels der mittelfrequenten Komponente kann der Kontrast bzw. das Kontrast-zu-Rausch-Verhältnis verbessert werden.

Gemäß einem Aspekt der Erfindung ist werden die mindestens zwei verschiedenen Frequenzkomponenten skaliert. Die mindestens zwei verschiedenen Frequenzkomponenten können gleich oder verschieden skaliert werden. Die skalierten Frequenzkomponenten können als zweite Bildkomponente verwendet werden. Vorteilhaft können die mindestens zwei Frequenzkomponenten aneinander angepasst skaliert werden. Vorteilhaft können die mindestens zwei Frequenzkomponenten an die erste Bildkomponente angepasst skaliert werden.

Erfindungsgemäß werden die erste Bildkomponente und die zweite Bildkomponente zum synthetischen Mammogramm rekombiniert. Durch Zusammenfügen bzw. Rekombinieren der ersten Bildkomponente und der zweiten Bildkomponente kann das synthetische Mammogramm erzeugt werden. Vorteilhaft wird besonders betont die Information des zugeordneten Projektionsdatensatzes in Form der ersten Bildkomponente gemeinsam mit zusätzlichen Informationen aus mehreren bzw. weiteren Projektionsdatensätzen in Form der zweiten Bildkomponente genutzt. Dadurch kann die Information basierend auf der verwendeten Patientendosis vorteilhaft für das Erzeugen des synthetischen Mammogramms verwendet werden.

Gemäß einem Aspekt der Erfindung wird ein Schritt der Nachverarbeitung auf das synthetische Mammogramm angewendet. Die Nachverarbeitung kann eine zusätzliche Filterung, Fensterung bzw. im Allgemeinen eine Anpassung der Darstellung umfassen. Vorteilhaft können von konventionellen digitalen Vollfeld-Mammographieaufnahmen bekannte Nachverarbeitungen auf das synthetische Mammogramm angewendet werden. Damit kann die Vergleichbarkeit weiterhin verbessert werden.

Gemäß einem Aspekt der Erfindung wird das synthetische Mammogramm mit einer früheren digitalen Vollfeld-Mammographieaufnahme, insbesondere mit gleichem Flavour, verglichen. Dies wird vorteilhaft durch das erfindungsgemäße synthetische Mammogramm ermöglicht. Der Vergleich findet insbesondere optisch durch einen Benutzer statt. Insbesondere können das synthetische Mammogramm und die frühere digitale Vollfeld-Mammographieaufnahme, insbesondere mit gleichem Flavour, nebeneinander dargestellt werden, entweder für eine Brustseite oder beide Brustseiten gleichzeitig. Dadurch kann der Vergleich vorteilhaft vereinfacht durchgeführt werden.

Gemäß einem Aspekt der Erfindung wird das synthetische Mammogramm aus mehreren erzeugten synthetischen Mammogrammen zum Vergleich mit einer früheren digitalen Vollfeld-Mammographieaufnahme ausgewählt. Werden mehrere synthetische Mammogramme erzeugt, so kann vom Anwender oder mittels eines Algorithmus ein für den Vergleich bevorzugt geeignetes synthetisches Mammogramm ausgewählt werden. Vorteilhaft kann der Vergleich einer früheren digitalen Vollfeld-Mammographieaufnahme mit einem synthetischen Mammogramm einer aktuellen Aufnahme vereinfacht werden. Die mehreren synthetischen Mammogramme können als sogenannte rotierende Mammogramme dargestellt werden, wobei durch das sogenannte Rotieren eine dreidimensionale Betrachtung möglich ist.

Gemäß einem Aspekt der Erfindung umfasst das synthetische Mammogramm eine CAD-Markierung bzw. CAD-Markierungen. Rechnergestützte Diagnoseunterstützung bzw. Bildanalyse kann in Form von sogenannten CAD-Markierungen (engl.: CAD marks, computer-aided diagnosis marks) bereitgestellt werden. Mittels Maschinenlernverfahren, insbesondere Deep-Learning-Verfahren, können Läsionen, besonders dichte Brustdichten oder Kalizfikationen markiert bzw. hervorgehoben werden. Die CAD-Markierung kann insbesondere innerhalb eines dreidimensionalen Tomosynthesevolumens bestimmt werden. Die CAD-Markierung kann im synthetischen Mammogramm, welches das betroffene Voxel der CAD-Markierung in der Projektion umfasst, angezeigt werden. Vorteilhaft ermöglicht die CAD-Markierung im synthetischen Mammogramm einen umfassenden Überblick über potentiell näher zu betrachtende Regionen innerhalb der Brust.

Die Erfindung betrifft ferner ein Mammographiesystem zum Durchführen eines Verfahrens nach einem der vorangehenden Ansprüche, aufweisend eine Aufnahmeeinheit und eine Erzeugungseinheit. Die Erzeugungseinheit kann bevorzugt von der Datenverarbeitungseinheit umfasst sein. Das Mammographiesystem kann ferner eine Einheit zum Ermitteln einer Durchschnittsintensitätsprojektion, eine Einheit zur Intensitätsanpassung, eine Einheit zur Grauwertverteilungsanpassung oder/und Streustrahlenkorrektur, eine Einheit zur kalkerhaltender Rauschfilterung, eine Einheit zum Ermitteln der Maximumsintensitätsprojektion, eine Einheit zur Frequenzzerlegung, eine Einheit zum Skalieren, eine Rekombinationseinheit und eine Aufprägeeinheit umfassen. Das Mammographiesystem kann vorteilhaft alle Schritte des erfindungsgemäßen Verfahrens durchführen. Das Mammographiesystem ist ein medizinisches Gerät. Es können alternativ auch andere medizinische Geräte, welche für Tomosyntheseverfahren geeignet sind, das erfindungsgemäße Verfahren anwenden.

Die Erfindung betrifft ferner ein Computerprogrammprodukt mit einem Computerprogramm, welches direkt in eine Speichereinrichtung einer Steuereinrichtung eines Mammographiesystems ladbar ist, mit Programmabschnitten, um alle Schritte des erfindungsgemäßen Verfahrens auszuführen, wenn das Computerprogramm in der Steuereinrichtung des Mammographiesystems ausgeführt wird.

Die Erfindung betrifft ferner ein computerlesbares Medium, auf welchem von einer Rechnereinheit einlesbare und ausführbare Programmabschnitte gespeichert sind, um alle Schritte des erfindungsgemäßen Verfahrens auszuführen, wenn die Programmabschnitte von der Recheneinheit ausgeführt werden. Die Recheneinheit kann bevorzugt von der Datenverarbeitungseinheit bzw. einer Prozessoreinheit umfasst sein.

Nachfolgend werden Ausführungsbeispiele der Erfindung anhand von Zeichnungen näher erläutert. Hierbei zeigt:
FIG 1 eine schematische Darstellung des erfindungsgemäßen Mammographiesystems in einer ersten Ausführungsform;
FIG 2 eine schematische Darstellung des erfindungsgemäßen Verfahrens;
FIG 3 eine schematische Darstellung der erfindungsgemäßen synthetischen Mammogramme in Gegenüberstellung zu konventionellen digitalen Vollfeld-Mammographieaufnahmen jeweils mit einer ausgewählten vordefinierten Bildeindruckseinstellung in einer ersten Ausführungsform;
FIG 4 eine schematische Darstellung der erfindungsgemäßen synthetischen Mammogramme in Gegenüberstellung zu konventionellen digitalen Vollfeld-Mammographieaufnahmen jeweils mit einer ausgewählten vordefinierten Bildeindruckseinstellung in einer zweiten Ausführungsform;
FIG 5 eine schematische Darstellung der erfindungsgemäßen synthetischen Mammogramme in Gegenüberstellung zu konventionellen digitalen Vollfeld-Mammographieaufnahmen jeweils mit einer ausgewählten vordefinierten Bildeindruckseinstellung in einer dritten Ausführungsform; und
FIG 6 eine schematische Darstellung des erfindungsgemäßen Mammographiesystems in einer zweiten Ausführungsform.

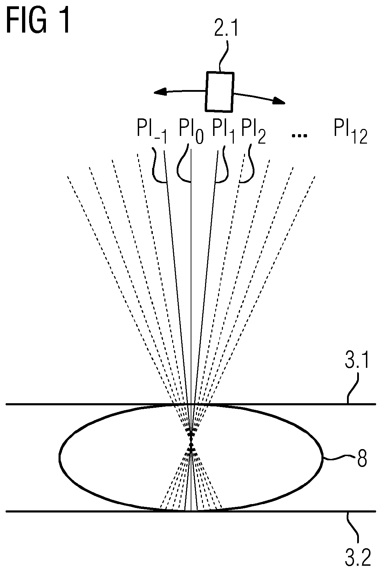

Die Fig. 1 zeigt eine beispielhafte Ausführung des erfindungsgemäßen Mammographiesystems in einer ersten Ausführungsform. Es wird eine Mehrzahl von Projektionsdatensätzen unter einer Mehrzahl von Projektionswinkeln PI_{-1,0,1,2,..., 12} aufgenommen. Die Röntgenquelle 2.1 wird dabei insbesondere entlang eines Radius um einen Punkt in der Brust 8 verfahren, wobei unter den Proj ektionswinkeln PI_{-1,0,1,2,..., 12} jeweils ein Projektionsdatensatz aufgenommen wird. Während der Aufnahme ist die Brust 8 eines Patienten als Untersuchungsobjekt zwischen einem oberen Kompressionselement 3.1 und einem unteren Kompressionselement 3.2 angeordnet.

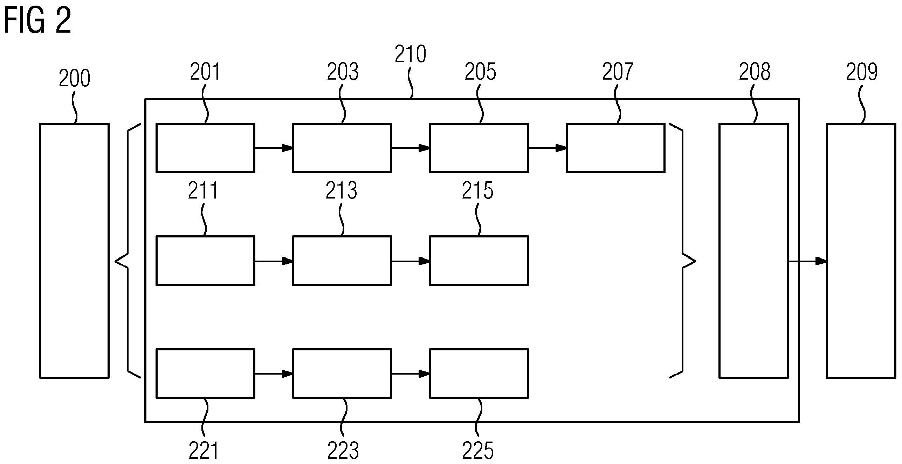

Die Fig. 2 zeigt eine beispielhafte Ausführung des erfindungsgemäßen Verfahrens zum Erzeugen eines synthetischen Mammogramms aufweisend die Schritte des Aufnehmens 200 und des Erzeugens 210. Die Fig. 2 zeigt insbesondere eine bevorzugte Reihenfolge der Schritte. Im Schritt des Aufnehmens wird eine Mehrzahl von Projektionsdatensätzen unter einer Mehrzahl von Projektionswinkeln aufgenommen. Im Schritt des Erzeugens wird mindestens ein synthetisches Mammogramm mit zu einer konventionellen digitalen Vollfeld-Mammographieaufnahme im Wesentlichen äquivalenten Bildeigenschaften basierend auf mehreren Projektionsdatensätzen erzeugt. In einer besonderen Ausführungsform werden mehrere synthetische Mammogramme für unterschiedliche Projektionswinkel erzeugt.

Im Schritt 201 wird eine Durchschnittsintensitätsprojektion (AIP) basierend auf der Mehrzahl von Projektionsdatensätzen als erste Bildkomponente ermittelt. Die Durchschnittsprojektion entspricht insbesondere einer Projektion, besonders bevorzugt der mittleren Projektion PI₀.

Werden mehrere synthetische Mammogramme erzeugt, so werden mehrere Projektionen jeweils als Durchschnittsprojektion verwendet, so dass für jede dieser Projektionen ein synthetisches Mammogramm erzeugt wird. Die maximale Anzahl der erzeugten synthetischen Mammogramme kann der Gesamtzahl der Projektionen entsprechen. Bevorzugt können beispielsweise für 17 von 25 Projektionen jeweils ein synthetisches Mammogramm erzeugt werden.

Auf die Durchschnittsintensitätsprojektion wird zumindest einer der folgenden Schritte angewendet: Intensitätsanpassung 203, Grauwertverteilungsanpassung 205 oder/und Streustrahlenkorrektur, und kalkerhaltender Rauschfilter 207.

Der Schritt des Erzeugens umfasst ein Ermitteln 211, 221 einer Maximumsintensitätsprojektion (MIP) basierend auf der Mehrzahl von Projektionsdatensätzen als zweite Bildkomponente. Die Maximumsintensitätsprojektion wird im Schritt 213 bzw. 223 in mindestens zwei verschiedene Frequenzkomponenten zerlegt. Die mindestens zwei verschiedenen Frequenzkomponenten umfassen im Schritt 213 hochfrequente Komponenten und im Schritt 223 mittelfrequente Komponenten. Die mindestens zwei verschiedenen Frequenzkomponenten werden in den Schritten 215, 225 skaliert.

Im Schritt des Rekombinierens 208 werden die erste Bildkomponente und die zweite Bildkomponente zum synthetischen Mammogramm rekombiniert. Das synthetische Mammogramm weist zu einer konventionellen digitalen Vollfeld-Mammographieaufnahme im Wesentlichen äquivalente Bildeigenschaften auf, wobei ein Schritt des Aufprägens 209 eine vordefinierte Bildeindruckseinstellung mittels einer Multifrequenzanpassung, einer Lookup-Tabellen-Anwendung, oder/und einer Fensterung umfassen kann. Es kann ferner ein weiterer Schritt der Nachverarbeitung auf das synthetische Mammogramm angewendet werden.

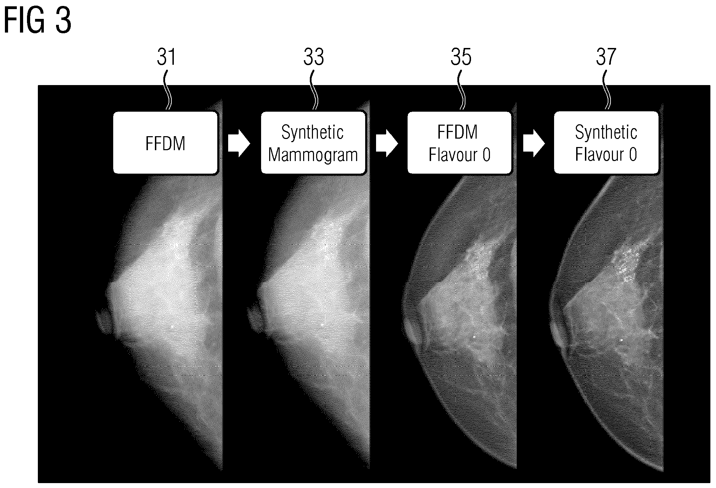

Die Fig. 3 zeigt eine beispielhafte Ausführung der erfindungsgemäßen synthetischen Mammogramme in Gegenüberstellung zu konventionellen digitalen Vollfeld-Mammographieaufnahmen jeweils mit einer ausgewählten vordefinierten Bildeindruckseinstellung in einer ersten Ausführungsform. Die Darstellung 31 zeigt eine konventionelle digitale Vollfeld-Mammographieaufnahme ohne vordefinierte Bildeindruckseinstellung. Die Darstellung 33 zeigt ein synthetisches Mammogramm mit zu einer konventionellen digitalen Vollfeld-Mammographieaufnahme im Wesentlichen äquivalenten Bildeigenschaften ohne vordefinierte Bildeindruckseinstellung. Die Darstellung 35 zeigt eine konventionelle digitale Vollfeld-Mammographieaufnahme mit vordefinierter Bildeindruckseinstellung der ersten Ausführungsform, insbesondere dem sogenannten Flavour 0. Die Darstellung 37 zeigt ein synthetisches Mammogramm mit vordefinierter Bildeindruckseinstellung der ersten Ausführungsform, insbesondere dem sogenannten Flavour 0.

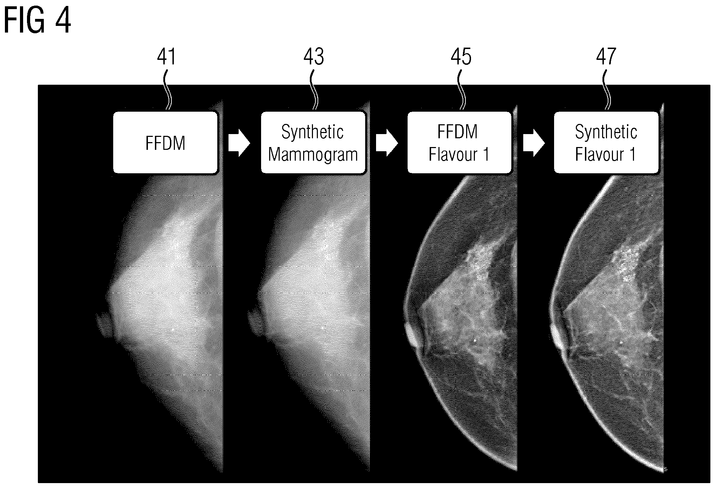

Die Fig. 4 zeigt eine beispielhafte Ausführung der erfindungsgemäßen synthetischen Mammogramme in Gegenüberstellung zu konventionellen digitalen Vollfeld-Mammographieaufnahmen jeweils mit einer ausgewählten vordefinierten Bildeindruckseinstellung in einer zweiten Ausführungsform. Die Darstellung 41 zeigt eine konventionelle digitale Vollfeld-Mammographieaufnahme ohne vordefinierte Bildeindruckseinstellung. Die Darstellung 43 zeigt ein synthetisches Mammogramm mit zu einer konventionellen digitalen Vollfeld-Mammographieaufnahme im Wesentlichen äquivalenten Bildeigenschaften ohne vordefinierte Bildeindruckseinstellung. Die Darstellung 45 zeigt eine konventionelle digitale Vollfeld-Mammographieaufnahme mit vordefinierter Bildeindruckseinstellung der zweiten Ausführungsform, insbesondere dem sogenannten Flavour 1. Die Darstellung 47 zeigt ein synthetisches Mammogramm mit vordefinierter Bildeindruckseinstellung der zweiten Ausführungsform, insbesondere dem sogenannten Flavour 1.

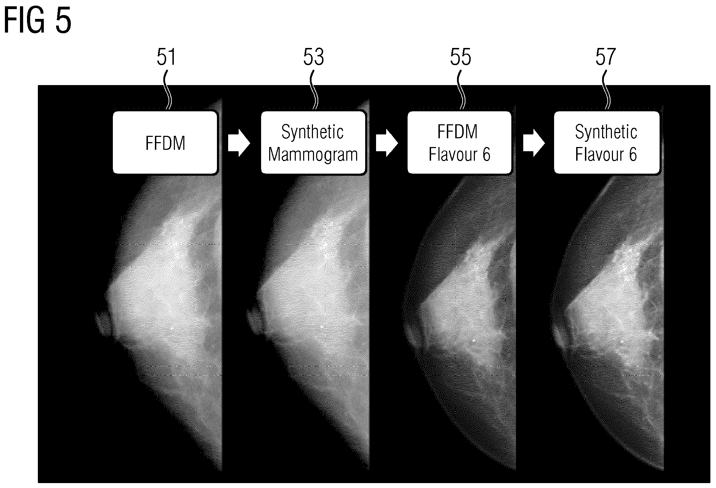

Die Fig. 5 zeigt eine beispielhafte Ausführung der erfindungsgemäßen synthetischen Mammogramme in Gegenüberstellung zu konventionellen digitalen Vollfeld-Mammographieaufnahmen jeweils mit einer ausgewählten vordefinierten Bildeindruckseinstellung in einer dritten Ausführungsform. Die Darstellung 51 zeigt eine konventionelle digitale Vollfeld-Mammographieaufnahme ohne vordefinierte Bildeindruckseinstellung. Die Darstellung 53 zeigt ein synthetisches Mammogramm mit zu einer konventionellen digitalen Vollfeld-Mammographieaufnahme im Wesentlichen äquivalenten Bildeigenschaften ohne vordefinierte Bildeindruckseinstellung. Die Darstellung 55 zeigt eine konventionelle digitale Vollfeld-Mammographieaufnahme mit vordefinierter Bildeindruckseinstellung der dritten Ausführungsform, insbesondere dem sogenannten Flavour 6. Die Darstellung 57 zeigt ein synthetisches Mammogramm mit vordefinierter Bildeindruckseinstellung der dritten Ausführungsform, insbesondere dem sogenannten Flavour 6.

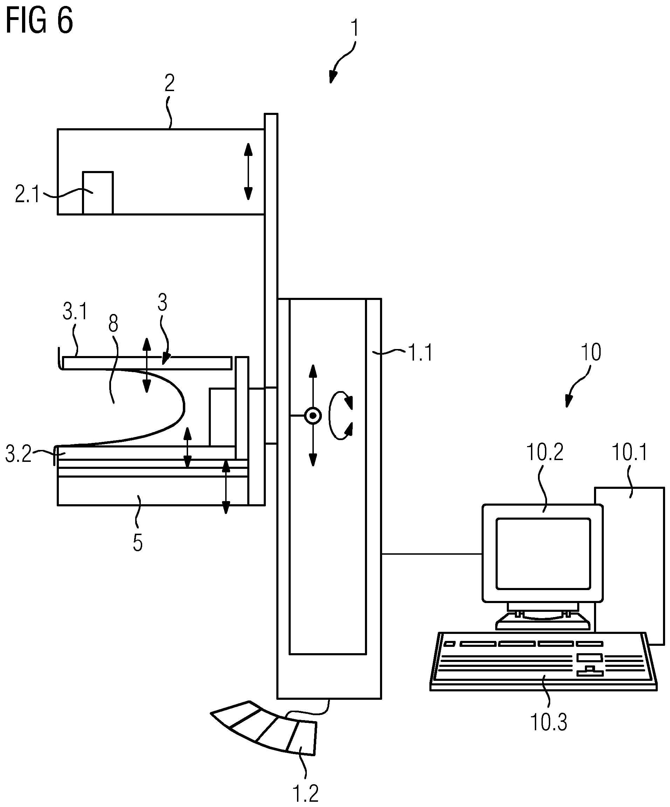

Die Fig. 6 zeigt eine beispielhafte Ausführung des erfindungsgemäßen Mammographiesystems 1 in einer zweiten Ausführungsform. Das Mammographiesystem 1 umfasst ein Stativ 1.1, an welchem das Röntgengehäuse 2 aufweisend die Röntgenquelle 2.1 und der Röntgendetektor 5 gemeinsam mit einer Kompressionseinheit 3 angeordnet sind. Insbesondere das Röntgengehäuse 2 ist bezüglich des Stativs 1.1 und des Röntgendetektors 5 sowie der Kompressionseinheit 3 rotierbar am Stativ 1.1 gelagert. Die Kompressionseinheit 3 umfasst ein oberes Kompressionselement 3.1 und ein unteres Kompressionselement 3.2, zwischen denen die Brust 8 eines Patienten angeordnet wird. Das Mammographiesystem 1 ist mit einer Datenverarbeitungseinheit 10 verbunden. Die Datenverarbeitungseinheit 10 umfasst mindestens eine Prozessoreinheit 10.1, eine Visualisierungseinheit 10.2 und eine Eingabeeinheit 10.3. Das Mammographiesystem 1 ist zumindest teilweise über einen Fußschalter 1.2 steuerbar.

Obwohl die Erfindung im Detail durch das bevorzugte Ausführungsbeispiel näher illustriert wurde, so ist die Erfindung nicht durch die offenbarten Beispiele eingeschränkt und andere Variationen können vom Fachmann hieraus abgeleitet werden, ohne den Schutzumfang der Erfindung, wie in den folgenden Ansprüchen definiert, zu verlassen.

## Patentansprüche

1. Verfahren zum Erzeugen eines synthetischen Mammogramms aufweisend die Schritte:
- Aufnehmen (200) einer Mehrzahl von Projektionsdatensätzen unter einer Mehrzahl von Projektionswinkeln (PI_{-1,0,1,2,...,12}),
- Erzeugen (210) mindestens eines synthetischen Mammogramms mit zu einer konventionellen digitalen Vollfeld-Mammographieaufnahme im Wesentlichen äquivalenter Bildeigenschaft basierend auf mehreren Projektionsdatensätzen,
- wobei der Schritt des Erzeugens (210) ein Ermitteln (201) einer Durchschnittsintensitätsprojektion basierend auf der Mehrzahl von Projektionsdatensätzen als erste Bildkomponente umfasst, wobei ein Projektionsdatensatz oder mehrere Projektionsdatensätze ausgewählt werden,
- auf die Durchschnittsintensitätsprojektion zumindest einer der folgenden Schritte angewendet wird:
- Intensitätsanpassung (203),
- Grauwertverteilungsanpassung (205),
- Streustrahlenkorrektur, und
- kalkerhaltender Rauschfilter (207),
- der Schritt des Erzeugens (210) ein Ermitteln (211, 221) einer Maximumsintensitätsprojektion basierend auf der Mehrzahl von Projektionsdatensätzen als zweite Bildkomponente umfasst, und
- die erste Bildkomponente und die zweite Bildkomponente zum synthetischen Mammogramm rekombiniert werden.

2. Verfahren nach Anspruch 1, ferner aufweisend den Schritt:
- Aufprägen (209) einer ausgewählten vordefinierten Bildeindruckseinstellung auf das synthetische Mammogramm.

3. Verfahren nach Anspruch 2, wobei die vordefinierte Bildeindruckseinstellung eine Multifrequenzanpassung, eine Lookup-Tabellen-Anwendung, oder/und eine Fensterung umfasst.

4. Verfahren nach einem der vorangehenden Ansprüche, wobei die Maximumsintensitätsprojektion in mindestens zwei verschiedene Frequenzkomponenten (213, 223) zerlegt wird.

5. Verfahren nach einem der vorangehenden Ansprüche, wobei die mindestens zwei verschiedenen Frequenzkomponenten hochfrequente Komponenten und mittelfrequente Komponenten umfassen.

6. Verfahren nach einem der Ansprüche 4 oder 5, wobei die mindestens zwei verschiedenen Frequenzkomponenten skaliert (215, 225) werden.

7. Verfahren nach einem der vorangehenden Ansprüche, wobei ein Schritt der Nachverarbeitung auf das synthetische Mammogramm angewendet wird.

8. Verfahren nach einem der vorangehenden Ansprüche, wobei das synthetische Mammogramm mit einer früheren digitalen Vollfeld-Mammographieaufnahme verglichen wird.

9. Verfahren nach Anspruch 8, wobei das synthetische Mammogramm aus mehreren erzeugten synthetischen Mammogrammen zum Vergleich mit einer früheren digitalen Vollfeld-Mammographieaufnahme ausgewählt wird.

10. Verfahren nach einem der vorangehenden Ansprüche, wobei das synthetische Mammogramm CAD-Markierungen umfasst.

11. Mammographiesystem (1) zum Durchführen eines Verfahrens nach einem der vorangehenden Ansprüche, aufweisend eine Aufnahmeeinheit (1.1, 2.1, 3, 5) und eine Erzeugungseinheit.

12. Computerprogrammprodukt mit einem Computerprogramm, welches direkt in eine Speichereinrichtung einer Steuereinrichtung eines Mammographiesystems (1) ladbar ist, mit Programmabschnitten, um alle Schritte eines Verfahrens nach einem der Ansprüche 1 bis 10 auszuführen, wenn das Computerprogramm in der Steuereinrichtung des Mammographiesystems (1) ausgeführt wird.

13. Computerlesbares Medium, auf welchem von einer Rechnereinheit einlesbare und ausführbare Programmabschnitte gespeichert sind, um alle Schritte eines Verfahrens nach einem der Ansprüche 1 bis 10 auszuführen, wenn die Programmabschnitte von der Recheneinheit ausgeführt werden.

## Claims

1. Method for generating a synthetic mammogram, having the steps:
- acquisition (200) of a plurality of projection data sets at a plurality of projection angles (PI_{-1,0,1,2,..., 12}),
- generation (210) of at least one synthetic mammogram with an image property essentially equivalent to a conventional full-field digital mammography acquisition based on several projection data sets,
- wherein the generation step (210) comprises a determination (201) of an average intensity projection based on the multiplicity of projection data sets as the first image component, wherein one projection data set or a plurality of projection data sets are selected,
- at least one of the following steps is applied to the average intensity projection:
- intensity adjustment (203),
- grey value distribution adjustment (205),
- scattered ray correction, and
- calcification-retaining noise filter (207),
- the generation step (210) comprises a determination (211, 221) of a maximum intensity projection based on the multiplicity of projection data sets as the second image component, and
- the first image component and the second image component are recombined to form the synthetic mammogram.

2. Method according to claim 1, further comprising the step:
- imprinting (209) of a selected predefined image impression setting onto the synthetic mammogram.

3. Method according to claim 2, wherein the predefined image impression setting comprises a multifrequency adjustment, a lookup table application, or/and a windowing.

4. Method according to one of the preceding claims, wherein the maximum intensity projection is broken down into at least two different frequency components (213, 223).

5. Method according to one of the preceding claims, wherein the at least two different frequency components comprise highfrequency components and medium-frequency components.

6. Method according to one of claims 4 or 5, wherein the at least two different frequency components are scaled (215, 225) .

7. Method according to one of the preceding claims, wherein a postprocessing step is applied to the synthetic mammogram.

8. Method according to one of the preceding claims, wherein the synthetic mammogram is compared with an earlier full-field digital mammography acquisition.

9. Method according to claim 8, wherein the synthetic mammogram is selected from several generated synthetic mammograms for comparison with an earlier full-field digital mammography acquisition.

10. Method according to one of the preceding claims, wherein the synthetic mammogram comprises CAD marks.

11. Mammography system (1) for carrying out a method according to one of the preceding claims, having an acquisition unit (1.1, 2.1, 3, 5) and a generation unit.

12. Computer program product with a computer program which can be loaded directly into a memory store of a control device of a mammography system (1), having program portions in order to carry out all steps of a method according to one of claims 1 to 10 when the computer program is executed in the control device of the mammography system (1).

13. Computer-readable medium on which program portions that can be read in and executed by a computer unit are stored, in order to carry out all steps of a method according to one of claims 1 to 10 when the program portions are executed by the computer unit.

## Revendications

1. Procédé de production d'un mammogramme synthétique comprenant les stades :
- enregistrement (200) d'une pluralité d'ensembles de données de projection sous une pluralité d'angles (PI_{-1,0,1,2,...,12}) de projection,
- production (210) d'au moins un mammogramme synthétique ayant une propriété d'image sensiblement équivalente à un enregistrement de mammographie numérique classique à plein champ reposant sur plusieurs ensembles de données de projection,
- dans lequel le stade de la production (210) comprend une détermination (201) d'une projection d'intensité moyenne reposant sur la pluralité d'ensembles de données de projection comme première composante d'image, dans lequel on choisit un ensemble de données de projection ou plusieurs ensembles de données de projection,
- on applique à la projection d'intensité moyenne au moins l'un des stades suivants :
- adaptation (203) d'intensité,
- adaptation (205) de la répartition de valeurs de gris,
- correction d'anti-diffusion, et
- filtrage (207) de bruit conservateur,
- le stade de la production (210) comprend une détermination (211, 221) d'une projection d'intensité maximum sur la base de la pluralité d'ensembles de données de projection comme deuxième composante d'image, et
- on recombine la première composante d'image et la deuxième composante d'image en le mammogramme synthétique.

2. Procédé suivant la revendication 1, comprenant en outre le stade :
- estampage (209) d'un réglage d'impression d'image sélectionné défini à l'avance sur le mammogramme synthétique.

3. Procédé suivant la revendication 2, dans lequel le réglage d'impression d'image défini à l'avance comprend une adaptation multifréquence, une application de table de consultation ou/et un fenestrage.

4. Procédé suivant l'une des revendications précédentes, dans lequel on décompose la projection d'intensité maximum en au moins deux composantes (213, 223) de fréquence différentes.

5. Procédé suivant l'une des revendications précédentes, dans lequel les au moins deux composantes de fréquence différentes comprennent des composantes de haute fréquence et des composantes de moyenne fréquence.

6. Procédé suivant l'une des revendications 4 ou 5, dans lequel on met à l'échelle (215, 225) les au moins deux composantes de fréquence différentes.

7. Procédé suivant l'une des revendications précédentes, dans lequel on applique un stade de traitement ultérieur au mammogramme synthétique.

8. Procédé suivant l'une des revendications précédentes, dans lequel on compare le mammogramme synthétique à un enregistrement de mammographie à plein champ numérique antérieure.

9. Procédé suivant la revendication 8, dans lequel on choisit le mammogramme synthétique composé de plusieurs mammogrammes synthétiques produits pour la comparaison à un enregistrement de mammographie à plein champ numérique antérieure.

10. Procédé suivant l'une des revendications précédentes, dans le mammogramme synthétique comprend des repérages CAD.

11. Système (1) de mammographie pour effectuer un procédé suivant l'une des revendications précédentes comportant une unité (1.1, 2.1, 3, 5) d'enregistrement et une unité de production.

12. Produit de programme d'ordinateur comprenant un programme d'ordinateur, qui peut être chargé directement dans un dispositif de mémoire d'un dispositif de commande d'un système (1) de mammographie, comprenant des parties de programme pour effectuer tous les stades d'un procédé suivant l'une des revendications 1 à 10, lorsque le programme d'ordinateur est exécuté dans le dispositif de commande du système (1) de mammographie.

13. Support, déchiffrable par ordinateur, sur lequel sont mises en mémoire des parties de programme déchiffrables et pouvant être réalisées par une unité informatique, afin d'effectuer tous les stades d'un procédé suivant l'une des revendications 1 à 10, lorsque les parties du programme sont exécutées par l'unité informatique.
